# EUROPEAN PATENT APPLICATION

(11) **EP 2 221 369 A1**
(43) Date of publication of application: **25.08.2010**
(21) Application number: 08847810.2
(22) Date of filing: 31.10.2008
(51) Int. Cl.: C12N 15/09, C07K 14/435, C12Q 1/68, G01N 33/50, G01N 33/53, G01N 33/68

(54) **MUTANT NUCLEIC ACID RELATED TO CHRONIC MYELOPROLIFERATIVE DISORDER, AND METHOD OF EVALUATING CHRONIC MYELOPROLIFERATIVE DISORDER**

(30) Priority: 07.11.2007 JP 2007289759
(71) Applicant: ARKRAY, Inc., Minami-ku Kyoto-shi Kyoto 601-8045 (JP); Nippon Medical School Foundation, Bunkyo-ku, Tokyo 113-8602 (JP)
(72) Inventor: YAMAGUCHI,Hiroki, Tokyo 113-8602 (JP); INAMI, Mitsuharu, Tokyo 113-8602 (JP)
(74) Representative: Jones, Elizabeth Louise
(86) International application number: PCT/JP2008/069953
(87) International publication number: WO 2009/060804

(57) **Abstract**

A new mutant gene related to the onset of CMPD, particularly, the new mutant gene related to the onset of CMPD in patients who develop CMPD despite of JAK2^{V617F}-negative, and an evaluation method for evaluating CMPD are provided. By detecting the following mutation in a JAK2 gene or an EPOR gene in a biological sample of human origin, the possibility of CMPD is evaluated. (a) a mutation from G at the 2116^{th} position to A in a base sequence of SEQ ID NO: 1, (b) a deletion of a nucleotide residue from the 2121^{st} to 2126^{th} position in a base sequence of SEQ ID NO: 1, (c) a mutation from C at the 1641^{st} position to G in a base sequence of SEQ ID NO: 4.

## Description

### Technical Field

The present invention relates to a new mutant nucleic acid related to chronic myeloproliferative disorder (CMPD) and an evaluation method of CMPD.

### Background Art

In recent years, with respect to a variety of diseases, diagnosis by gene analysis has been tried. According to this method, for example, it can be evaluated whether the onset of the diseases is confirmed or not, and the susceptibility to the disease, by analyzing the presence or absence of a specific mutation with respect to a specific gene. Therefore, with respect to every disease, identification of a genetic mutation that shows a relationship with the onset thereof has been conducted.

With respect to chronic myeloproliferative disorder (CMPD) such as polycythemia vera (PV), essential thrombocythemia (ET), idiopathic myelofibrosis (IMF), and the like, a specific mutation in a JAK2 (Janus kinase 2) gene has been reported as a mutation related to the diseases (nonpatent documents 1 to 4). This mutation is a mutation (JAK2^{V617F}) substituting valine (V) at the 617^{th} position in JAK2 protein for phenylalanine (F). This mutation exists in a JH2 region of the JAK2 gene, and in which guanine at the 73^{rd} position in exon12, i.e., guanine at the 2343^{rd} position in a sequence of open reading frame (ORF) with without intron, is substituted for thymine. Further, this mutation is said to cause activation of the JAK-STAT pathway, which leads to an increase in blood-forming cells, and indeed, in Western countries, it is reported that 65% to 99 % of PV patients, 23% to 72% of ET patients, and 35% to 57% of IMF patients have a mutation of JAK2^{V617F}.

However, there are cases in which some patients develop CMPD, although they show JAK2^{V617F}-negative. Especially, with respect to PV patients and ET patients, for example, it is reported that clinical characteristics such as thrombotic event, hemorrhagic event, and transformation to acute leukemia are different between Japanese and Occidental, and further, the onset rate of CMPD in JAK2^{V617F}-positive patients of Japanese is lower than that of Occidental.
[Nonpatent document 1] Tefferi A. Hematology Am Soc Hematol Educ Program. 2006; 240-245.
[Nonpatent document 2] Baxter EJ, Scott LM, Campbell PJ, et al. Lancet. 2005; 365: 1054-1061.
[Nonpatent document 3] Kralovics R, Passamonti F, Buser AS, et al. N Eng1 J Med. 2005; 352: 1779-1790.
[Nonpatent document 4] Levine RL, Wadleigh M, Cools J, et al. Cancer Cell. 2005; 7: 387-397

### Disclosure of Invention

Hence, the present invention is intended to provide a new mutant gene related to CMPD, especially, a new mutant gene related to CMPD in JAK2^{V617F}-negative patients, and a CMPD evaluation method that evaluates CMPD by the detection of the same.

In order to achieve the aforementioned object, a mutant nucleic acid of the present invention is composed of at least one nucleic acid selected from the group consisting of the following (i), (ii), and (iii):
(i) a nucleic acid composed of a JAK2 gene including any one of the following mutations (a) and (b) or a fragment thereof including the mutation
   (a) a mutation from G at the 2116^{th} position to A in a base sequence of SEQ ID NO: 1
   (b) a deletion of a nucleotide residue from the 2121^{st} to 2126^{th} position in a base sequence of SEQ ID NO: 1
(ii) a nucleic acid composed of an EPOR gene including the following mutation (c) or a fragment thereof including the mutation (c) a mutation from C at the 1641^{st} position to G in a base sequence of SEQ ID NO: 4
(iii) a nucleic acid composed of a base sequence complementary to at least one of the nucleic acid of (i) and the nucleic acid of (ii).

A mutant polypeptide of the present invention is composed of any one of the following polypeptides (I), and (II).
(I) a polypeptide composed of JAK2 protein including any one of the following mutations (A) and (B) or a fragment thereof including the mutation
   (A) a mutation from Arg at the 541^{st} position to Lys in an amino-acid sequence of SEQ ID NO: 2
   (B) a deletion of Glu at the 543^{rd} position and Asp at the 544^{th} position in an amino-acid sequence of SEQ ID NO: 2
(II) a polypeptide composed of EPOR protein including the following mutation (C) or a fragment thereof including the mutation
   (C) a mutation from Pro at the 502^{nd} position to Arg in an amino-acid sequence of SEQ ID NO: 5

A marker of CMPD of the present invention includes a mutant nucleic acid of the present invention or a mutant polypeptide of the present invention.

A CMPD evaluation method of the present invention is an evaluation method for evaluating the possibility of CMPD by detecting at least one mutation of a nucleic acid and a polypeptide in a biological sample. The evaluation method includes any one of the following processes (X) and (Y):
(X) a process for detecting the presence or absence of a mutant nucleic acid of the present invention in the sample
(Y) a process for detecting the presence or absence of a mutant polypeptide of the present invention in the sample

An evaluation kit of the present invention is an evaluation kit for evaluating CMPD and includes at least one of
a probe which is a polynucleotide including at least one mutation selected from the group consisting of the (a), (b), and (c), or a polynucleotide composed of a base sequence complementary thereto and which can hybridize to a mutant nucleic acid of the present invention, and
a primer which is a polynucleotide including at least one mutation selected from the group consisting of the (a), (b), and (c), or a polynucleotide composed of a base sequence complementary thereto and which can hybridize to the mutant nucleic acid of the present invention. Further, the evaluation kit of the present invention includes an antibody, an antigen thereof is a mutant polypeptide of the present invention.

The inventors found, as a result of earnest study, a new mutation related to CMPD in a human JAK2 (Janus kinase 2) gene and a human EPOR (erythropoietin receptor) gene, and reached the present invention. Because a mutant nucleic acid of the present invention and a mutant polypeptide that is an expression product thereof are related to CMPD, they can be used for a marker of CMPD. Further, detection of them allows evaluation of the possibility of the onset of CMPD. Especially, because these mutations were detected from conventionally reported JAK2^{V617F}-negative CMPD patients, the evaluation of the possibility of the onset of CMPD became possible with respect to JAK2^{V617F}-negative patients whose onset of CMPD could not be evaluated by detection of JAK2^{V617F}, by detecting a mutant nucleic acid and a mutant polypeptide of the present invention. Therefore, for example, by detecting JAK2^{V617F} in combination with a mutation of the present invention, further accurate evaluation can be achieved. Accordingly, the present invention is very useful technology in a clinical medical field, and the like. In addition, a mutation in the present invention and a relationship between the mutation and diseases are found by the inventors for the first time.

### Brief Description of Drawings

[FIG. 1] FIG.1 is a schematic diagram of a JAK2 gene and a view showing mutation sites in Example 1 of the present invention.
[FIG. 2] FIG.2 is a schematic diagram of an EPOR gene and a view showing mutation sites in Example 1 of the present invention.

### Description of the Invention

In the present invention, examples of chronic myeloproliferative disorder (CMPD) include myelofibrosis with myeloid metaplasia (MMM) such as idiopathic myelofibrosis (IMF), primary myelofibrosis (PMF) and the like; erythrocytosis such as polycythemia rubra vera, polycythemia vera (PV), secondary absolute erythrocytosis, secondary absolute polycythemia, stress erythrocytosis, stresspolycythemia, and the like; polycythemia; essential thrombocythemia (ET); and hyper eosinophilic syndrome (HES); and the like.

### < mutant nucleic acid and marker>

As described above, a mutant gene of the present invention is composed of at least one nucleic acid selected from the group consisting of the following (i), (ii), and (iii):
(i) a nucleic acid composed of a JAK2 gene including any one of the following mutations (a) and (b) or a fragment thereof including the mutation
   (a) a mutation from G at the 2116^{th} position to A in a base sequence of SEQ ID NO: 1
   (b) a deletion of a nucleotide residue from the 2121^{st} to 2126^{th} position in a base sequence of SEQ ID NO: 1
(ii) a nucleic acid composed of an EPOR gene including the following mutation (c) or a fragment thereof including the mutation
   (c) a mutation from C at the 1641^{st} position to G in a base sequence of SEQ ID NO: 4
(iii) a nucleic acid composed of a base sequence complementary to at least one of the nucleic acid of (i) and the nucleic acid of (ii).

Mutations (a), (b), and (c) of a gene newly found by the inventors are (a) a substitution mutation of a human JAK2 gene, (b) a deletion mutation of a human JAK2 gene, and (c) a substitution mutation of an EPOR gene, respectively. A base sequence of SEQ ID NO: 1 is a DNA sequence (sense strand) in a JAK2 gene and is a cDNA sequence excluding an intron. In a base sequence of SEQ ID NO: 1, a sequence in which a base t is a base u is a mature mRNA sequence in which an intron of a JAK2 gene is spliced. In a base sequence of SEQ ID NO: 1, a region from the 495^{th} to 3893^{rd} position is CDS. A base sequence of the CDS is shown in SEQ ID NO: 3. A sequence of a JAK2 gene is, for example, registered at NCBI under the accession No. NM_004972. On the other hand, a base sequence of SEQ ID NO: 4 is a DNA sequence (sense strand) of an EPOR gene and is a cDNA sequence excluding an intron. In the base sequence of SEQ ID NO: 1, a sequence in which a base t is a base u is a mature mRNA sequence in which an intron of an EPOR gene is spliced. In the base sequence of SEQ ID NO: 4, a region from the 137^{th} to 1663^{rd} position is CDS. A base sequence of the CDS is shown in SEQ ID NO: 6. In SEQ ID NO: 6, a base sequence from the 1^{st} to 72^{nd} position is a coding sequence at a signaling site of EPOR protein, and a base sequence from the 73^{rd} to 1527^{th} position is a coding sequence of mature EPOR protein. A sequence of an EPOR gene is, for example, registered at NCBI under the accession No. NM_000121.

When a mutant nucleic acid of the present invention is translated, Arg at the 541^{st} position in a full-length amino-acid sequence of JAK2 protein is substituted for Lys by the mutation (a), and Glu at the 543^{rd} position and Asp at the 544^{th} position in a full-length amino-acid sequence of JAK2 protein are deleted by the mutation (b). Further, when a mutant nucleic acid of the present invention is translated, Pro at the 502^{nd} position in a full-length amino-acid sequence of EPOR protein is substituted for Arg by the mutation (c). Hereinafter, the mutation (a) is also referred to as [R541K] or [JAK2^{R541K}], the mutation (b) is also referred to as [E543-D544 del] or [JAK2^{E543-D544 del}], and the mutation (c) is also referred to as [P478R] or [EPOR^{P48R}].

In (a), (b), and (c), base sequences of SEQ ID NO: 1 and SEQ ID NO: 4 are a description for specifying a new mutation site in a JAK2 gene or an EPOR gene, and a sequence of a mutant nucleic acid of the present invention is not limited to each full-length sequence. Therefore, the mutant nucleic acid of the present invention may be a full-length gene composed of a base sequence of SEQ ID NO: 1 or SEQ ID NO: 4 or a polynucleotide composed of a partial sequence thereof as long as it has any one of the mutations described above. Specific examples thereof include CDS shown in SEQ ID NO: 3 and SEQ ID NO: 6, and a polynucleotide composed of a partial sequence thereof. A fragment of (i) includes, for example, in a base sequence of SEQ ID NO: 1, a polynucleotide composed of a contiguous 5 to 10,000 base sequence including a base (A) at the 2116^{th} position, preferably a polynucleotide composed of a contiguous 8 to 1,000 base sequence, and more preferably a polynucleotide composed of a contiguous 10 to 100 base sequence. Further, a fragment of (ii) includes, for example, in a base sequence of SEQ ID NO: 4, a polynucleotide composed of a contiguous 5 to 10,000 base sequence including a deletion of a nucleotide residue from the 2121^{st} to 2126^{th} position, preferably a polynucleotide composed of a contiguous 8 to 1,000 base sequence, and more preferably a polynucleotide composed of a contiguous 10 to 100 base sequence. Furthermore, the mutant nucleic acid of the present invention may be, for example, a nucleic acid composed of an antisense strand of a JAK2 gene or a fragment thereof, or a nucleic acid composed of an antisense strand of an EPOR gene or a fragment thereof. In this instance, as shown in (iii), the mutant nucleic acid of the present invention includes a nucleic acid composed of a base sequence complementary to at least one of a nucleic acid of (i) and a nucleic acid (ii).

Further, as long as it has any one of the mutations described above, the mutant nucleic acid of the present invention may not only be a gene composed of a base sequence of SEQ ID NO:1, a gene composed of a base sequence which does not include an intron sequence showing in SEQ ID NO:3, and their fragments, a gene composed of a base sequence of SEQ ID NO:4, a gene composed of a base sequence which does not include an intron sequence shown in SEQ ID NO:6, and their fragments, but also be a gene including an intron, its fragment, or a nucleic acid composed of a base sequence complementary thereto, for example.

Furthermore, the mutant nucleic acid of the present invention is not limited to DNA, and also may be RNA, for example. When the mutant nucleic acid of the present invention is mutant RNA, for example, as described above, [t] may be [u], in a base sequences of SEQ ID NO: 1, 3, 4, or 6.

In addition, the mutant nucleic acid may be DNA or RNA that is originally contained in the sample, for example. Also, DNA may be DNA synthesized from DNA that is originally contained in the sample by a nucleic acid amplification method such as PCR and the like, or may be cDNA synthesized from RNA (total RNA, mRNA, etc.) that is originally contained in the sample by Reverse Transcription PCR (RT-PCR), for example.

Next, a CMPD marker of the present invention includes the mutant nucleic acid of the present invention. As described above, because a mutation in the mutant nucleic acid of the present invention shows relationship with the onset of CMPD, the mutant nucleic acid of the present invention can be used as a marker of CMPD. Therefore, as will be described later, for example, the onset and the possibility of the onset of CMPD can be evaluated by detecting the presence or absence of the mutant nucleic acid.

### <mutant polypeptide>

As described above, a mutant polypeptide of the present invention is composed of any one of the following polypeptides (I), and (II). This mutant polypeptide is, for example, an expression product of the mutant nucleic acid of the present invention.
(I) a polypeptide composed of JAK2 protein including any one of the following mutations (A) and (B) or a fragment thereof including the mutation
   (A) a mutation from Arg at the 541^{st} position to Lys in an amino-acid sequence of SEQ ID NO: 2
   (B) a deletion of Glu at the 543^{rd} position and Asp at the 544^{th} position in an amino-acid sequence of SEQ ID NO: 2
(II) a polypeptide composed of EPOR protein including the following mutation (C) or a fragment thereof including the mutation
   (C) a mutation from Pro at the 502^{nd} position to Arg in an amino-acid sequence of SEQ ID NO: 5

Mutations (A), (B), and (C) of a polypeptide, newly found by the inventors are (A) a substitution mutation of human JAK2 protein, (B) a deletion mutation of human JAK2 protein, and (C) a substitution mutation of EPOR protein, respectively. The amino-acid sequence of SEQ ID NO: 2 is an amino-acid sequence of JAK2 protein, and this sequence is, for example, registered at NCBI under the accession No. NM_004972. The amino-acid sequence of SEQ ID NO: 5 is an amino-acid sequence of EPOR protein, and the sequence is, for example, registered at NCBI under the accession No. NM_000121. In the SEQ ID NO: 5, an amino-acid sequence from the 1^{st} to 24^{th} position is a signal sequence of EPOR protein, and an amino-acid sequence from the 25^{th} to 508^{th} position is a sequence of mature EPOR protein excluding a signal sequence.

In (A), (B), and (C), amino-acid sequences of SEQ ID NO: 2 and SEQ ID NO: 5 are a description for specifying a mutation site, and a sequence of the mutant polypeptide of the present invention is not limited to each full-length sequence. Therefore, as long as it has any one of the mutations described above, the mutant polypeptide of the present invention may be a full-length polypeptide (protein) composed of an amino-acid sequence of SEQ ID NO: 2 or SEQ ID NO: 5 or may be a polypeptide fragment composed of a partial sequence thereof. Specific examples thereof include mature EPOR protein from the 25^{th} to 508^{th} position of SEQ ID NO: 5 and a polypeptide fragment composed of a partial sequence thereof.

Next, a CMPD marker of the present invention includes the mutant polypeptide of the present invention. As described above, because the mutation in the mutant polypeptide of the present invention shows a relationship with the onset of CMPD, the mutant polypeptide of the present invention can be used as a marker of CMPD. Therefore, as will be described later, the onset and the possibility of the onset of CMPD can be evaluated by detecting the presence or absence of the mutant polypeptide.

### <CMPD evaluation method >

A CMPD evaluation method of the present invention is an evaluation method for evaluating the possibility of CMPD by detecting at least one mutation of a nucleic acid and a polypeptide in a biological sample. The evaluation method includes at least one of the following processes (X) and (Y):
(X) a process for detecting the presence or absence of a mutant nucleic acid of the present invention in the sample
(Y) a process for detecting the presence or absence of a mutant polypeptide of the present invention in the sample

In the present invention, any one of the mutant nucleic acid and the mutant polypeptide of the present invention may be detected. When the mutant nucleic acid or the mutant polypeptide of the present invention is detected, for example, it may be judged as CMPD or susceptibility to CMPD. When the mutant nucleic acid or the mutant polypeptide of the present invention is not detected, for example, it may be judged as having low susceptibility to CMPD or no susceptibility to CMPD. In addition, the method for detecting the mutant nucleic acid of the present invention and the method for detecting the mutant polypeptide of the present invention are not particularly limited and the conventionally known method of detecting a mutation can be adopted.

Examples of a sample to be subjected to the present invention include a hemocyte sample such as a leukocyte cell and the like; a whole blood sample; a bone-marrow sample; and the like, although it is not particularly limited. Further, the sample is, for example, preferably a biological sample of human or a biological sample of mammals excluding human.

The processes (X) and (Y) respectively are described below. However, they are mere examples and do not limit the present invention.

### (Embodiment 1)

As the first embodiment, an example of the process (X), i.e., a process of detecting the presence or absence of the mutant nucleic acid of the present invention in the sample is described.

The presence or absence of a mutant nucleic acid in the sample can be detected by detecting at least one mutation selected from the group consisting of the following (a), (b), and (c) with respect to the nucleic acid in the sample, for example.
(a) a mutation from G at the 2116^{th} position to A in a base sequence of SEQ ID NO: 1
(b) a deletion of a nucleotide residue from the 2121^{st} to 2126^{th} position in a base sequence of SEQ ID NO: 1
(c) a mutation from C at the 1641^{st} position to G in a base sequence of SEQ ID NO: 4

The detection of the mutation may be conducted with respect to any one of them, two or more of them, or all of them. Further, the mutations of the (a) and (b) preferably are detected together.

The mutation can be detected, by hybridizing a nucleic acid in the sample with a probe that can hybridize specifically to a region including the mutation, for example. In this instance, when the hybridization between the probe and the nucleic acid in the sample is confirmed, it can be judged as having a mutation, and when the hybridization is not confirmed, it can be judged as not having a mutation. An example of the probe includes a polynucleotide including any one of the mutations in the (a), (b), and (c) or a polynucleotide composed of a base sequence complementary thereto, and which can hybridize to a mutant nucleic acid including the mutation. Examples of this kind of detection method include an Invader method, a Tm analysis method, and the like.

Further, the mutation can be detected by, for example, conducting a nucleic acid amplification reaction in which a nucleic acid in the sample is used as a template with a primer that can hybridize specifically to a region including the mutation. In this instance, when an amplification product is obtained by a nucleic acid amplification reaction, it can be judged as having a mutation, and when an amplification product is not obtained, it can be judged as not having a mutation. An example of the primer includes a polynucleotide including at least one of the mutations selected from the group (a), (b), and (c) or a polynucleotide composed of a base sequence complementary thereto, and which can hybridize to the mutant nucleic acid including the mutation. Examples of this kind of detection method include an ASP (allele specific primer) - PCR method, and the like. In this instance, as the primer, preferably a primer which has the mutant base or a base complementary to the mutant base at the 3' region, especially at the 3' end or the first base or the second base from the 3' end is used.

In addition, examples of the method include a Direct Sequencing method, a Pyrosequencing method, a Denaturing HPLC method, and the like. In the Direct Sequencing method and the Pyrosequencing method, with respect to a nucleic acid in the sample, a region including a site where a target mutation occurs is amplified and the entire base sequence of an amplification product is analyzed, and in the Denaturing HPLC method, HPLC is performed with respect to the amplification product in a temperature gradient column and the presence or absence of a mutation is detected from an elution time.

The nucleic acid amplification method is not limited at all, and examples thereof include a PCR method, a Nucleic acid sequence based amplification (NASBA) method, a Transcription-mediated amplification (TMA) method, a Strand Displacement Amplification (SDA) method, and the like. In addition, the conditions of a nucleic acid amplification reaction are not particularly limited and can be conducted by the conventionally known method.

### (Embodiment 2)

As the second embodiment, an example of the process (Y), i.e., a process of detecting the presence or absence of a mutant polypeptide of the present invention in the sample is described.

The presence or absence of a mutant polypeptide in the sample can be detected by detecting at least one mutation selected from the group consisting of the following (A), (B), and (C) with respect to a polypeptide in the sample, for example.
(A) a mutation from Arg at the 541^{st} position to Lys in an amino-acid sequence of SEQ ID NO: 2
(B) a deletion of Glu at the 543^{rd} position and Asp at the 544^{th} position in an amino-acid sequence of SEQ ID NO: 2
(C) a mutation from Pro at the 502^{nd} position to Arg in an amino-acid sequence of SEQ ID NO: 5

The detection of the mutation may be conducted with respect to any one of them, two or more of them, or all of them. Further, the mutations of the (A) and (B) are preferably detected together.

A mutation of a polypeptide can be detected, by an antigen-antibody reaction between the antibody and a polypeptide in the sample, with an antibody, whose antigen is a mutant polypeptide of the present invention, for example. In this instance, when the antigen-antibody reaction between the antibody and a polypeptide in the sample is confirmed, it can be judged as having a mutation, and when the antigen-antibody reaction is not confirmed, it can be judged as not having a mutation. The type of an antibody is not particularly limited, and a monoclonal antibody, a polyclonal antibody, and the like can be used. The technique of the antigen-antibody reaction is not limited, and immunoblotting technique and immunohistochemical technique, and the like can be used.

### <CMPD evaluation kit>

A CMPD evaluation kit of the present invention includes, for example, a first evaluation kit that can be used for detecting the mutant nucleic acid of the present invention, and a second evaluation kit which can be used for detecting the mutant polypeptide of the present invention.

The first evaluation kit of the present invention, as described above, is an evaluation kit for evaluating CMPD and includes at least one of a probe that is a polynucleotide including at least one mutation selected from the group consisting of the (a), (b), and (c), or a polynucleotide composed of a base sequence complementary thereto, and that can hybridize specifically thereto, and a primer which is a polynucleotide including at least one mutation selected from the group consisting of the (a), (b), and (c), or a polynucleotide composed of the base sequence complementary thereto, and that can hybridize thereto. Further, the second evaluation kit of the present invention, as described above, includes an antibody, and an antigen thereof is the mutant polypeptide of the present invention. By using these evaluation kits of the present invention, the CMPD evaluation method of the present invention can be conducted simply and easily. In addition, a primer, a probe and, an antibody are similar to as those described above.

The configuration of the first evaluation kit of the present invention is not particularly limited and, for example, further may include a primer, DNA polymerase, dNTPs, various additives, which are required for the nucleic acid amplification. Further, the configuration of the second evaluation kit of the present invention is also not particularly limited and, for example, further may include a secondary antibody for detecting the antibody, and enzyme and substrate for detecting a label added to an antibody. In addition the evaluation kit of the present invention may further include an instruction manual.

### Examples

Next, examples of the present invention are described. However, the present invention is not limited by the following examples.

### [Example 1]

Genomic DNAs were prepared from total of 130 patients, PV patients (n=52), ET patients (n=55), IMF patients (n=3), CMP D-unclassified (CMPD-U) patents (n=15), and hyper eosinophilic syndrome (HES) patients (n=5) who are diagnosed by the heretofore known standard. Also genomic DNAs were prepared from healthy subjects. The genomic DNAs of the patients were extracted from buffy coats of bone marrow obtained from the patients and the genomic DNAs of the healthy subjects were extracted from peripheral blood granulocytes obtained from the healthy subjects, respectively, by using QIAamp DNA Mini Kit (trade name, manufactured by QIAGEN GmbH). The genomic DNAs of the healthy subjects were used as a normal control, and analysis of mutations and polymorphisms was conducted by using a mutation analysis apparatus (manufactured by ARKRAY, Inc.).

With respect to the genomic DNAs of 130 patients, for analyzing the mutation of JAK2^{V611F}, an ASP-PCR method and a Direct Sequencing method were conducted in accordance with a common procedure (Baxter EJ, et al. Acquired mutation of the tyrosine kinase JAK2 in human myeloproliferative disorders. Lancet. 2005; 365: 1054-1061). Further, PCR products were introduced to a pCR (registered trademark) 2.1-TOPO vector by using a TOPO TA cloning kit (trade name, manufactured by Invitrogen Corporation) and a sequence of the PCR products were confirmed by a sequence of the resultant recombinant plasmid.

By the analysis, JAK2^{V617F} was confirmed in 31 out of 52 PV patients (59.6%), 28 out of 55 ET patients (50.9%), 1 out of 3 IMF patients (33.3%), 5 out of 15 CMPD-U patients (33.3%), and 1 out of 5 HES patients (20%). From the result, as described above, it was found that the onset rate of JAK2^{V617F}-positive PV patients of Japanese (59.6%) is lower than that of Occidental (65∼99%).

Next, with respect to genomic DNAs of the JAK2^{V617F}-negative CMPD patients, analysis of a mutation was conducted. As a result, the mutation (JAK2^{E543-D544 del}) in which G was substituted for A at a nucleotide residue at the 2116^{th} position of SEQ ID NO: 1 (at the 1621^{st} position in CDS of SEQ ID NO: 3) (JAK2^{R541K}), and a nucleotide residue (total of 6 bp) at the 2121^{st} to 2126^{th} position of SEQ ID NO: 1 (at the 1625^{th} to 1630^{th} position in CDS of SEQ ID NO: 3) was deleted, was found from JAK2^{V617F}-negative PV patients.

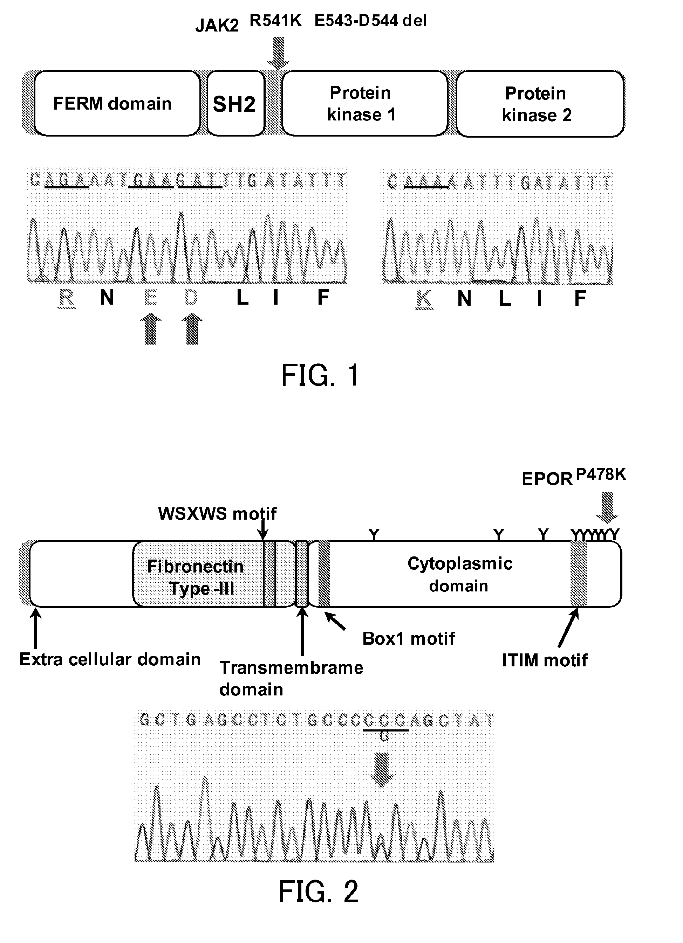

FIG. 1 shows a schematic diagram of a partial region of a JAK2 gene and a sequence result. In FIG. 1, arrows indicate positions of the mutation (JAK2^{R541K E543-D544 del}) which are newly found. In FIG. 1, the left-sided base sequence is a sequence of wild-type allele, and the right-sided base sequence is a sequence of variant allele. In addition, the underlined codons are codons in which mutation occurs. As shown in FIG. 1, JAK2^{R541K} and JAK2^{E543-D544 del}are new mutations existing between SH2 domain and Protein kinase1 domain in the exon 12 (when considering exon 1 and exon 2 of a nontranscribed region, it is in the exon 14) of a JAK2 gene. This mutation was adjacent to the deletion mutation that already has been reported (Scott LM, et al. JAK2 exon 12 mutations in polycythemia vera and idiopathic erythrocytosis. N Engl J Med. 2007; 356: 459-468.). In addition, with respect to an amino-acid residue at the 541^{st} position of SEQ ID NO: 2, arginine (R) is substituted for lysine (K) when G at the 2116^{th} position of SEQ ID NO: 1 is substituted for A. These mutations, JAK2^{R541K} and JAK2^{E543-D544 del}, were not detected from the healthy subjects.

With respect to one patient whom a mutation was detected, clinical characteristics are shown in the following Table 1. As a result, as shown in the following Table 1, it was found that although this patient was a PV patient of JAK2^{V617F}-negative, the typical clinical characteristics of PV were confirmed in the same manner as the patients of the JAK2^{V617F}-positive.

**[Table 1]**

| | patient | age | gender | WBC | RBC | Hb | Ht | PLT | diagnosis | cytogenetical analysis | JAK2V617F mutation |
|---|---|---|---|---|---|---|---|---|---|---|---|
| JAK2^{R541KE543-D544 del} | 1 | 52 | F | 6900 | 857 | 19 | 59.5 | 43 | PV | 46,XX | (-) |
| EPOR^{P478R} | 2 | 75 | M | 15700 | 276 | 4.9 | 19.8 | 525 | ET | 46,XX | (-) |
| | | | | | | | | | | del(11)(q21q23) | (-) |
| | 3 | 39 | F | 6900 | 432 | 13 | 38 | 103 | ET | 46,XX | (-) |

| | | | | | | | | | | | |
|---|---|---|---|---|---|---|---|---|---|---|---|
| WBC : white blood cell counts (/µ l) RBC : red blood cell counts (×10⁴/µ l) Hb : hemoglobin concentration (mg/100ml) Ht : hematocrit value (%) PLT : platelet counts (×10⁴/µl) | | | | | | | | | | | |

Next, with respect to the CMPD patients, the entire sequence of an EPOR gene was analyzed. As a result, the mutation (EPOR^{P502R}) in which C was substituted for G at a nucleotide residue at the 1641^{st} position of SEQ ID NO: 3. (at the 1505^{th} position in CDS of SEQ ID NO: 6) was confirmed from 2 ET patients who were JAK2^{V617F}-negative.

FIG. 2 shows a schematic diagram of a partial region of an EPOR gene and a sequence result. The thick arrows indicate the position of the newly found mutation (EPOR^{P478K}) and Y shows position of a tyrosine residue at a gene. The base sequence in FIG. 2 is a sequence of wild-type allele, underlined codons are codons in which mutation occurs and CCC mutates to CGC. As shown in FIG. 2, with respect to EPOR^{P502R}, proline is substituted for arginine. This mutation is a new mutation existing in the exon 8 which is a negative adjustment region of cytoplasmic domain of an EPOR gene. In addition, with respect to an amino-acid residue at the 502^{nd} position of SEQ ID NO: 4, proline is substituted for arginine when C at the 1641^{st} position of SEQ ID NO: 3 is substituted for G. This mutation, EPOR^{P502R}, was not detected from the healthy subjects.

As described above, because JAK2^{R541K}, JAK2^{E543-D544 del}, and EPOR^{P502R} were found from CMPD patients of JAK2^{V617F}-negative, the evaluation of the possibility of the onset of CMPD by detection of these mutations with respect to a specimen of AK2^{V617F}-negative which could not be evaluated, can be achieved. In addition, because these mutations were found from CMPD patients of JAK2^{V617F}- negative, these mutations are considered as not corresponding to a secondary mutation.

### Industrial Applicability

As described above, because the mutant nucleic acid of the present invention and the mutant polypeptide, the expression product of the mutant nucleic acid of the present invention, are related to CMPD, they can be used as a marker of CMPD. Further, by detecting them, evaluation of the possibility of the onset of CMPD can be achieved. Particularly, because these mutations were detected from conventionally reported CMPD patients of JAK2^{V617F}-negative, by detecting the mutant nucleic acid and the mutant polypeptide of the present invention, evaluation of the possibility of the onset of CMPD could be achieved with respect to JAK2^{V617F}-negative patients who could not be evaluated by detection of JAK2^{V617F}. Therefore, the present invention is very useful technology in a clinical medical field, and the like. In addition, the mutation of the present invention and the relation between the mutations and diseases are found by the inventors for the first time.

### Sequence listing

TF08040-01. ST25. txt

## Claims

1. A mutant nucleic acid composed of at least one nucleic acid selected from the group consisting of the following (i), (ii), and (iii):
(i) a nucleic acid composed of a JAK2 gene including any one of the following mutations (a) and (b) or a fragment thereof including the mutation
(a) a mutation from G at the 2116^{th} position to A in a base sequence of SEQ ID NO: 1
(b) a deletion of a nucleotide residue from the 2121^{st} to 2126^{th} position in a base sequence of SEQ ID NO: 1
(ii) a nucleic acid composed of an EPOR gene including the following mutation (c) or a fragment thereof including the mutation
(c) a mutation from C at the 1641^{st} position to G in a base sequence of SEQ ID NO: 4.
(iii) a nucleic acid composed of a base sequence complementary to at least one of the nucleic acid of (i) and the nucleic acid of (ii).

2. A mutant polypeptide composed of any one of the following polypeptides (I) and (II):
(I) a polypeptide composed of JAK2 protein including any one of
the following mutations (A) and (B) or a fragment thereof including the mutation
(A) a mutation from Arg at the 541^{st} position to Lys in an amino-acid sequence of SEQ ID NO: 2
(B) a deletion of Glu at the 543^{rd} position and Asp at the 544^{th} position in an amino-acid sequence of SEQ ID NO: 2
(II) a polypeptide composed of EPOR protein including the following mutation (C) or a fragment thereof including the mutation
(C) a mutation from Pro at the 502^{nd} position to Arg in an amino-acid sequence of SEQ ID NO: 5.

3. A marker of CMPD including a mutant nucleic acid according to claim 1.

4. A marker of CMPD including a mutant polypeptide according to claim 2.

5. An evaluation method for evaluating the possibility of CMPD by detecting at least one mutation of a nucleic acid and a polypeptide in a biological sample, wherein
the evaluation method includes at least one process of the following (X) and (Y):
(X) a process for detecting the presence or absence of a mutant nucleic acid according to claim 1 in the sample
(Y) a process for detecting the presence or absence of a mutant polypeptide according to claim 2 in the sample.

6. The evaluation method according to claim 5, wherein, in the process (X), with respect to the nucleic acid in the sample, at least one mutation selected from the group consisting of the following (a), (b), and (c) is detected
(a) a mutation from G at the 2116^{th} position to A in a base sequence of SEQ ID NO: 1
(b) a deletion of a nucleotide residue from the 2121^{st} to 2126^{th} position in a base sequence of SEQ ID NO: 1
(c) a mutation from C at the 1641^{st} position to G in a base sequence of SEQ ID NO: 4.

7. The evaluation method according to claim 5, wherein, in the process (X), the mutation is detected with a probe which is a polynucleotide including at least one mutation selected from the group consisting of the (a), (b), and (c) or a polynucleotide composed of a base sequence complementary thereto, and that can hybridize to the mutant nucleic acid according to claim 1, by hybridization of the probe and a nucleic acid of the sample.

8. The evaluation method according to claim 5, wherein, in the process (X), the mutation is detected with a primer that is a polynucleotide including at least one mutation selected from the group consisting of the (a), (b), and (c) or a polynucleotide composed of a base sequence complementary thereto, and that can hybridize to the mutant nucleic acid according to claim 1, by a nucleic acid amplification reaction in which a nucleic acid in the sample is used as a template.

9. The evaluation method according to claim 8, wherein the primer has the mutant base or a base complementary to the mutant base at 3' region.

10. The evaluation method according to claim 5, wherein, in the process (Y), with respect to the polypeptide in the sample, at least one mutation selected from the group consisting of the following (A), (B), and (C) is detected
(A) a mutation from Arg at the 541^{st} position to Lys in an amino-acid sequence of SEQ ID NO: 2
(B) a deletion of Glu at the 543^{rd} position and Asp at the 544^{th} position in an amino-acid sequence of SEQ ID NO: 2
(C) a mutation from Pro at the 502^{nd} position to Arg in an amino-acid sequence of SEQ ID NO: 5.

11. The evaluation method according to claim 5, wherein, in the process (Y), the mutation is detected with an antibody, an antigen thereof is the mutant polypeptide according to claim 2, by an antigen-antibody reaction of the antibody and the polypeptide in the sample.

12. The evaluation method according to claim 5, wherein the sample is a biological sample in which a JAK2^{V617F} mutation is negative.

13. An evaluation kit for evaluating CMPD, wherein the evaluation kit includes any one of
a probe which is a polynucleotide including at least one mutation selected from the group consisting of the following (a), (b), and (c) or a polynucleotide composed of a base sequence complementary thereto, and can hybridize to a mutant nucleic acid according to claim 1 and
a primer which is a polynucleotide including at least one mutation selected from the group consisting of the following (a), (b), and (c) or a polynucleotide composed of a base sequence complementary thereto, and can hybridize to the mutant nucleic acid according to claim 1.

14. An evaluation kit for evaluating CMPD, wherein the evaluation kit includes an antibody, and an antigen thereof is a mutant polypeptide according to claim 2.


SEQUENCE LISTING

<110>  Arkray, Inc.

<120>  Mutated Nucleic Acid Related to Chronic Myeloproliferative Disease and
Method for Evaluation of the Disease

<130>  T07088-01

<150>  JP 2007/289759
<151>  2007-11-07

<160>  6

<170>  PatentIn version 3.1

<210>  1
<211>  5097
<212>  DNA
<213>  Homo sapiens

<220>
<221>  CDS
<222>  (495)..(3893)
<223>

<220>
<221>  mutation
<222>  (2121)..(2126)
<223>  6mers is deleted

<220>
<221>  mutation
<222>  (2116)..(2116)
<223>  g is substituted by a

<400>  1
ctgcaggaag gagagaggaa gaggagcaga agggggcagc agcggacgcc gctaacggcc      60
tccctcggcg ctgacaggct gggccggcgc ccggctcgct tgggtgttcg cgtcgccact     120
tcggcttctc ggccggtcgg gcccctcggc ccgggcttgc ggcgcgcgtc ggggctgagg     180
gctgctgcgg cgcagggaga ggcctggtcc tcgctgccga gggatgtgag tgggagctga     240
gcccacactg gagggccccc gagggcccag cctggaggtc gttcagagcc gtgcccgccc     300
cggggcttcg cagaccttga cccgccgggt aggagccgcc cctgcgggct cgagggcgcg     360
ctctggtcgc ccgatctgtg tagccggttt cagaagcagg caacaggaac aagatgtgaa     420
ctgtttctct tctgcagaaa aagaggctct tcctcctcct cccgcgacgg caaatgttct     480
gaaaaagact ctgc atg gga atg gcc tgc ctt acg atg aca gaa atg gag      530
gga aca tcc acc tct tct ata tat cag aat ggt gat att tct gga aat      578
gcc aat tct atg aag caa ata gat cca gtt ctt cag gtg tat ctt tac      626
cat tcc ctt ggg aaa tct gag gca gat tat ctg acc ttt cca tct ggg      674
gag tat gtt gca gaa gaa atc tgt att gct gct tct aaa gct tgt ggt      722
atc aca cct gtg tat cat aat atg ttt gct tta atg agt gaa aca gaa      770
agg atc tgg tat cca ccc aac cat gtc ttc cat ata gat gag tca acc      818
agg cat aat gta ctc tac aga ata aga ttt tac ttt cct cgt tgg tat      866
tgc agt ggc agc aac aga gcc tat cgg cat gga ata tct cga ggt gct      914
gaa gct cct ctt ctt gat gac ttt gtc atg tct tac ctc ttt gct cag      962
tgg cgg cat gat ttt gtg cac gga tgg ata aaa gta cct gtg act cat      1010
gaa aca cag gaa gaa tgt ctt ggg atg gca gtg tta gat atg atg aga      1058
ata gcc aaa gaa aac gat caa acc cca ctg gcc atc tat aac tct atc      1106
agc tac aag aca ttc tta cca aaa tgt att cga gca aag atc caa gac      1154
tat cat att ttg aca agg aag cga ata agg tac aga ttt cgc aga ttt      1202
att cag caa ttc agc caa tgc aaa gcc act gcc aga aac ttg aaa ctt      1250
aag tat ctt ata aat ctg gaa act ctg cag tct gcc ttc tac aca gag      1298
aaa ttt gaa gta aaa gaa cct gga agt ggt cct tca ggt gag gag att      1346
ttt gca acc att ata ata act gga aac ggt gga att cag tgg tca aga      1394
ggg aaa cat aaa gaa agt gag aca ctg aca gaa cag gat tta cag tta      1442
tat tgc gat ttt cct aat att att gat gtc agt att aag caa gca aac      1490
caa gag ggt tca aat gaa agc cga gtt gta act atc cat aag caa gat      1538
ggt aaa aat ctg gaa att gaa ctt agc tca tta agg gaa gct ttg tct      1586
ttc gtg tca tta att gat gga tat tat aga tta act gca gat gca cat      1634
cat tac ctc tgt aaa gaa gta gca cct cca gcc gtg ctt gaa aat ata      1682
caa agc aac tgt cat ggc cca att tcg atg gat ttt gcc att agt aaa      1730
ctg aag aaa gca ggt aat cag act gga ctg tat gta ctt cga tgc agt      1778
cct aag gac ttt aat aaa tat ttt ttg act ttt gct gtc gag cga gaa      1826
aat gtc att gaa tat aaa cac tgt ttg att aca aaa aat gag aat gaa      1874
gag tac aac ctc agt ggg aca aag aag aac ttc agc agt ctt aaa gat      1922
ctt ttg aat tgt tac cag atg gaa act gtt cgc tca gac aat ata att      1970
ttc cag ttt act aaa tgc tgt ccc cca aag cca aaa gat aaa tca aac      2018
ctt cta gtc ttc aga acg aat ggt gtt tct gat gta cca acc tca cca     2066
aca tta cag agg cct act cat atg aac caa atg gtg ttt cac aaa atc     2114
aga aat gaa gat ttg ata ttt aat gaa agc ctt ggc caa ggc act ttt     2162
aca aag att ttt aaa ggc gta cga aga gaa gta gga gac tac ggt caa     2210
ctg cat gaa aca gaa gtt ctt tta aaa gtt ctg gat aaa gca cac aga     2258
aac tat tca gag tct ttc ttt gaa gca gca agt atg atg agc aag ctt     2306
tct cac aag cat ttg gtt tta aat tat gga gta tgt gtc tgt gga gac     2354
gag aat att ctg gtt cag gag ttt gta aaa ttt gga tca cta gat aca     2402
tat ctg aaa aag aat aaa aat tgt ata aat ata tta tgg aaa ctt gaa     2450
gtt gct aaa cag ttg gca tgg gcc atg cat ttt cta gaa gaa aac acc     2498
ctt att cat ggg aat gta tgt gcc aaa aat att ctg ctt atc aga gaa     2546
gaa gac agg aag aca gga aat cct cct ttc atc aaa ctt agt gat cct     2594
ggc att agt att aca gtt ttg cca aag gac att ctt cag gag aga ata     2642
cca tgg gta cca cct gaa tgc att gaa aat cct aaa aat tta aat ttg     2690
gca aca gac aaa tgg agt ttt ggt acc act ttg tgg gaa atc tgc agt     2738
gga gga gat aaa cct cta agt gct ctg gat tct caa aga aag cta caa     2786
ttt tat gaa gat agg cat cag ctt cct gca cca aag tgg gca gaa tta      2834
gca aac ctt ata aat aat tgt atg gat tat gaa cca gat ttc agg cct      2882
tct ttc aga gcc atc ata cga gat ctt aac agt ttg ttt act cca gat      2930
tat gaa cta tta aca gaa aat gac atg tta cca aat atg agg ata ggt      2978
gcc cta ggg ttt tct ggt gcc ttt gaa gac cgg gat cct aca cag ttt      3026
gaa gag aga cat ttg aaa ttt cta cag caa ctt ggc aag ggt aat ttt      3074
ggg agt gtg gag atg tgc cgg tat gac cct cta cag gac aac act ggg      3122
gag gtg gtc gct gta aaa aag ctt cag cat agt act gaa gag cac cta      3170
aga gac ttt gaa agg gaa att gaa atc ctg aaa tcc cta cag cat gac      3218
aac att gta aag tac aag gga gtg tgc tac agt gct ggt cgg cgt aat      3266
cta aaa tta att atg gaa tat tta cca tat gga agt tta cga gac tat      3314
ctt caa aaa cat aaa gaa cgg ata gat cac ata aaa ctt ctg cag tac      3362
aca tct cag ata tgc aag ggt atg gag tat ctt ggt aca aaa agg tat      3410
atc cac agg gat ctg gca acg aga aat ata ttg gtg gag aac gag aac      3458
aga gtt aaa att gga gat ttt ggg tta acc aaa gtc  ttg cca caa gac      3506
aaa gaa tac tat aaa gta aaa gaa cct ggt gaa agt ccc ata ttc      3551
tgg tat gct cca gaa tca ctg aca gag agc aag ttt tct gtg gcc      3596
tca gat gtt tgg agc ttt gga gtg gtt ctg tat gaa ctt ttc aca      3641
tac att gag aag agt aaa agt cca cca gcg gaa ttt atg cgt atg      3686
att ggc aat gac aaa caa gga cag atg atc gtg ttc cat ttg ata      3731
gaa ctt ttg aag aat aat gga aga tta cca aga cca gat gga tgc      3776
cca gat gag atc tat atg atc atg aca gaa tgc tgg aac aat aat      3821
gta aat caa cgc ccc tcc ttt agg gat cta gct ctt cga gtg gat      3866
caa ata agg gat aac atg gct gga tga aagaaatgac cttcattctg        3913
agaccaaagt agatttacag aacaaagttt tatatttcac attgctgtgg actattatta   3973
catatatcat tattatataa atcatgatgc tagccagcaa agatgtgaaa atatctgctc   4033
aaaactttca aagtttagta agtttttctt catgaggcca ccagtaaaag acattaatga   4093
gaattcctta gcaaggattt tgtaagaagt ttcttaaaca ttgtctgtta acatcactct   4153
tgtctggcaa aagaaaaaaa atagactttt tcaactcagc tttttgagac ctgaaaaaat   4213
tattatgtaa attttgcaat gttaaagatg cacagaatat gtatgtatag tttttaccac   4273
agtggatgta taataccttg gcatcttgtg tgatgtttta cacacatgag ggctggtgtt   4333
cattaatact gttttctaat ttttccatag ttaatctata attaattact tcactataca   4393
aacaaattaa gatgttcaga taattgaata agtacctttg tgtccttgtt catttatatc   4453
gctggccagc attataagca ggtgtatact tttagcttgt agttccatgt actgtaaata   4513
tttttcacat aaagggaaca aatgtctagt tttatttgta taggaaattt ccctgaccct   4573
aaataataca ttttgaaatg aaacaagctt acaaagatat aatctatttt attatggttt   4633
cccttgtatc tatttgtggt gaatgtgttt tttaaatgga actatctcca aatttttcta 4693
agactactat gaacagtttt cttttaaaat tttgagatta agaatgccag gaatattgtc 4753
atcctttgag ctgctgactg ccaataacat tcttcgatct ctgggattta tgctcatgaa 4813
ctaaatttaa gcttaagcca taaaatagat tagattgttt tttaaaaatg gatagctcat 4873
taagaagtgc agcaggttaa gaattttttc ctaaagactg tatatttgag gggtttcaga 4933
attttgcatt gcagtcatag aagagattta tttccttttt agaggggaaa tgaggtaaat 4993
aagtaaaaaa gtatgcttgt taattttatt caagaatgcc agtagaaaat tcataacgtg 5053
tatctttaag aaaaatgagc atacatctta aatcttttca atta 5097

<210> 2
<211> 1132
<212> PRT
<213> Homo sapiens

<400> 2

<210> 3
<211> 3399
<212> DNA
<213> Homo sapiens

<400> 3
   atgggaatgg cctgccttac gatgacagaa atggagggaa catccacctc ttctatatat 60
   cagaatggtg atatttctgg aaatgccaat tctatgaagc aaatagatcc agttcttcag 120
   gtgtatcttt accattccct tgggaaatct gaggcagatt atctgacctt tccatctggg 180
   gagtatgttg cagaagaaat ctgtattgct gcttctaaag cttgtggtat cacacctgtg 240
   tatcataata tgtttgcttt aatgagtgaa acagaaagga tctggtatcc acccaaccat 300
   gtcttccata tagatgagtc aaccaggcat aatgtactct acagaataag attttacttt 360
   cctcgttggt attgcagtgg cagcaacaga gcctatcggc atggaatatc tcgaggtgct 420
   gaagctcctc ttcttgatga ctttgtcatg tcttacctct ttgctcagtg gcggcatgat 480
   tttgtgcacg gatggataaa agtacctgtg actcatgaaa cacaggaaga atgtcttggg 540
   atggcagtgt tagatatgat gagaatagcc aaagaaaacg atcaaacccc actggccatc 600
   tataactcta tcagctacaa gacattctta ccaaaatgta ttcgagcaaa gatccaagac 660
   tatcatattt tgacaaggaa gcgaataagg tacagatttc gcagatttat tcagcaattc 720
   agccaatgca aagccactgc cagaaacttg aaacttaagt atcttataaa tctggaaact 780
   ctgcagtctg ccttctacac agagaaattt gaagtaaaag aacctggaag tggtccttca 840
   ggtgaggaga tttttgcaac cattataata actggaaacg gtggaattca gtggtcaaga 900
   gggaaacata aagaaagtga gacactgaca gaacaggatt tacagttata ttgcgatttt 960
   cctaatatta ttgatgtcag tattaagcaa gcaaaccaag agggttcaaa tgaaagccga 1020
   gttgtaacta tccataagca agatggtaaa aatctggaaa ttgaacttag ctcattaagg 1080
   gaagctttgt ctttcgtgtc attaattgat ggatattata gattaactgc agatgcacat 1140
   cattacctct gtaaagaagt agcacctcca gccgtgcttg aaaatataca aagcaactgt 1200
   catggcccaa tttcgatgga ttttgccatt agtaaactga agaaagcagg taatcagact 1260
   ggactgtatg tacttcgatg cagtcctaag gactttaata aatatttttt gacttttgct 1320
   gtcgagcgag aaaatgtcat tgaatataaa cactgtttga ttacaaaaaa tgagaatgaa 1380
   gagtacaacc tcagtgggac aaagaagaac ttcagcagtc ttaaagatct tttgaattgt 1440
   taccagatgg aaactgttcg ctcagacaat ataattttcc agtttactaa atgctgtccc 1500
   ccaaagccaa aagataaatc aaaccttcta gtcttcagaa cgaatggtgt ttctgatgta 1560
   ccaacctcac caacattaca gaggcctact catatgaacc aaatggtgtt tcacaaaatc 1620
   agaaatgaag atttgatatt taatgaaagc cttggccaag gcacttttac aaagattttt 1680
   aaaggcgtac gaagagaagt aggagactac ggtcaactgc atgaaacaga agttctttta 1740
   aaagttctgg ataaagcaca cagaaactat tcagagtctt tctttgaagc agcaagtatg 1800
   atgagcaagc tttctcacaa gcatttggtt ttaaattatg gagtatgtgt ctgtggagac 1860
   gagaatattc tggttcagga gtttgtaaaa tttggatcac tagatacata tctgaaaaag 1920
   aataaaaatt gtataaatat attatggaaa cttgaagttg ctaaacagtt ggcatgggcc 1980
   atgcattttc tagaagaaaa cacccttatt catgggaatg tatgtgccaa aaatattctg 2040
   cttatcagag aagaagacag gaagacagga aatcctcctt tcatcaaact tagtgatcct 2100
   ggcattagta ttacagtttt gccaaaggac attcttcagg agagaatacc atgggtacca 2160
   cctgaatgca ttgaaaatcc taaaaattta aatttggcaa cagacaaatg gagttttggt 2220
   accactttgt gggaaatctg cagtggagga gataaacctc taagtgctct ggattctcaa 2280
   agaaagctac aattttatga agataggcat cagcttcctg caccaaagtg ggcagaatta 2340
   gcaaacctta taaataattg tatggattat gaaccagatt tcaggccttc tttcagagcc 2400
   atcatacgag atcttaacag tttgtttact ccagattatg aactattaac agaaaatgac 2460
   atgttaccaa atatgaggat aggtgcccta gggttttctg gtgcctttga agaccgggat 2520
   cctacacagt ttgaagagag acatttgaaa tttctacagc aacttggcaa gggtaatttt 2580
   gggagtgtgg agatgtgccg gtatgaccct ctacaggaca acactgggga ggtggtcgct 2640
   gtaaaaaagc ttcagcatag tactgaagag cacctaagag actttgaaag ggaaattgaa 2700
   atcctgaaat ccctacagca tgacaacatt gtaaagtaca agggagtgtg ctacagtgct 2760
   ggtcggcgta atctaaaatt aattatggaa tatttaccat atggaagttt acgagactat 2820
   cttcaaaaac ataaagaacg gatagatcac ataaaacttc tgcagtacac atctcagata 2880
   tgcaagggta tggagtatct tggtacaaaa aggtatatcc acagggatct ggcaacgaga 2940
   aatatattgg tggagaacga gaacagagtt aaaattggag attttgggtt aaccaaagtc 3000
   ttgccacaag acaaagaata ctataaagta aaagaacctg gtgaaagtcc catattctgg 3060
   tatgctccag aatcactgac agagagcaag ttttctgtgg cctcagatgt ttggagcttt 3120
   ggagtggttc tgtatgaact tttcacatac attgagaaga gtaaaagtcc accagcggaa 3180
   tttatgcgta tgattggcaa tgacaaacaa ggacagatga tcgtgttcca tttgatagaa 3240
   cttttgaaga ataatggaag attaccaaga ccagatggat gcccagatga gatctatatg 3300
   atcatgacag aatgctggaa caataatgta aatcaacgcc cctcctttag ggatctagct 3360
   cttcgagtgg atcaaataag ggataacatg gctggatga 3399

<210> 4
<211> 1849
<212> DNA
<213> Homo sapiens

<220>
<221> CDS
<222> (137)..(1663)
<223>

<220>
<221> mutation
<222> (1641)..(1641)
<223> c is substituted by g

<400> 4
acttagaggc gcctggtcgg gaagggcctg gtcagctgcg tccggcggag gcagctgctg 60

acccagctgt ggactgtgcc gggggcgggg gacggagggg caggagccct gggctccccg 120

tggcgggggc tgtatc atg gac cac ctc ggg gcg tcc ctc tgg ccc cag gtc 172
Met Asp His Leu Gly Ala Ser Leu Trp Pro Gln Val
1               5                   10

ggc tcc ctt tgt ctc ctg ctc gct ggg gcc gcc tgg gcg ccc ccg cct 220
Gly Ser Leu Cys Leu Leu Leu Ala Gly Ala Ala Trp Ala Pro Pro Pro
        15                  20                  25

aac ctc ccg gac ccc aag ttc gag agc aaa gcg gcc ttg ctg gcg gcc 268
Asn Leu Pro Asp Pro Lys Phe Glu Ser Lys Ala Ala Leu Leu Ala Ala
    30                  35                  40

cgg ggg ccc gaa gag ctt ctg tgc ttc acc gag cgg ttg gag gac ttg 316
Arg Gly Pro Glu Glu Leu Leu Cys Phe Thr Glu Arg Leu Glu Asp Leu
45                  50                  55                  60

gtg tgt ttc tgg gag gaa gcg gcg agc gct ggg gtg ggc ccg ggc aac 364
Val Cys Phe Trp Glu Glu Ala Ala Ser Ala Gly Val Gly Pro Gly Asn
                65                  70                  75

tac agc ttc tcc tac cag ctc gag gat gag cca tgg aag ctg tgt cgc 412
Tyr Ser Phe Ser Tyr Gln Leu Glu Asp Glu Pro Trp Lys Leu Cys Arg
            80                  85                  90

ctg cac cag gct ccc acg gct cgt ggt gcg gtg cgc ttc tgg tgt tcg 460
Leu His Gln Ala Pro Thr Ala Arg Gly Ala Val Arg Phe Trp Cys Ser
        95                  100                 105

ctg cct aca gcc gac acg tcg agc ttc gtg ccc cta gag ttg cgc gtc 508
Leu Pro Thr Ala Asp Thr Ser Ser Phe Val Pro Leu Glu Leu Arg Val
    110                 115                 120

aca gca gcc tcc ggc gct ccg cga tat cac cgt gtc atc cac atc aat 556

Thr Ala Ala Ser Gly Ala Pro Arg Tyr His Arg Val Ile His Ile Asn
125                 130                 135                 140

gaa gta gtg ctc cta gac gcc ccc gtg ggg ctg gtg gcg cgg ttg gct      604
Glu Val Val Leu Leu Asp Ala Pro Val Gly Leu Val Ala Arg Leu Ala
                145                 150                 155

gac gag agc ggc cac gta gtg ttg cgc tgg ctc ccg ccg cct gag aca      652
Asp Glu Ser Gly His Val Val Leu Arg Trp Leu Pro Pro Pro Glu Thr
            160                 165                 170

ccc atg acg tct cac atc cgc tac gag gtg gac gtc tcg gcc ggc aac      700
Pro Met Thr Ser His Ile Arg Tyr Glu Val Asp Val Ser Ala Gly Asn
        175                 180                 185

ggc gca ggg agc gta cag agg gtg gag atc ctg gag ggc cgc acc gag      748
Gly Ala Gly Ser Val Gln Arg Val Glu Ile Leu Glu Gly Arg Thr Glu
    190                 195                 200

tgt gtg ctg agc aac ctg cgg ggc cgg acg cgc tac acc ttc gcc gtc      796
Cys Val Leu Ser Asn Leu Arg Gly Arg Thr Arg Tyr Thr Phe Ala Val
205                 210                 215                 220

cgc gcg cgt atg gct gag ccg agc ttc ggc ggc ttc tgg agc gcc tgg      844
Arg Ala Arg Met Ala Glu Pro Ser Phe Gly Gly Phe Trp Ser Ala Trp
                225                 230                 235

tcg gag cct gtg tcg ctg ctg acg cct agc gac ctg gac ccc ctc atc      892
Ser Glu Pro Val Ser Leu Leu Thr Pro Ser Asp Leu Asp Pro Leu Ile
            240                 245                 250

ctg acg ctc tcc ctc atc ctc gtg gtc atc ctg gtg ctg ctg acc gtg      940
Leu Thr Leu Ser Leu Ile Leu Val Val Ile Leu Val Leu Leu Thr Val
        255                 260                 265

ctc gcg ctg ctc tcc cac cgc cgg gct ctg aag cag aag atc tgg cct      988
Leu Ala Leu Leu Ser His Arg Arg Ala Leu Lys Gln Lys Ile Trp Pro
    270                 275                 280

ggc atc ccg agc cca gag agc gag ttt gaa ggc ctc ttc acc acc cac     1036
Gly Ile Pro Ser Pro Glu Ser Glu Phe Glu Gly Leu Phe Thr Thr His
285                 290                 295                 300

aag ggt aac ttc cag ctg tgg ctg tac cag aat gat ggc tgc ctg tgg     1084
Lys Gly Asn Phe Gln Leu Trp Leu Tyr Gln Asn Asp Gly Cys Leu Trp
                305                 310                 315

tgg agc ccc tgc acc ccc ttc acg gag gac cca cct gct tcc ctg gaa     1132
Trp Ser Pro Cys Thr Pro Phe Thr Glu Asp Pro Pro Ala Ser Leu Glu
            320                 325                 330

gtc ctc tca gag cgc tgc tgg ggg acg atg cag gca gtg gag ccg ggg     1180
Val Leu Ser Glu Arg Cys Trp Gly Thr Met Gln Ala Val Glu Pro Gly
        335                 340                 345

aca gat gat gag ggc ccc ctg ctg gag cca gtg ggc agt gag cat gcc     1228
Thr Asp Asp Glu Gly Pro Leu Leu Glu Pro Val Gly Ser Glu His Ala
    350                 355                 360

cag gat acc tat ctg gtg ctg gac aaa tgg ttg ctg ccc cgg aac ccg     1276
Gln Asp Thr Tyr Leu Val Leu Asp Lys Trp Leu Leu Pro Arg Asn Pro

365                 370                 375                 380

ccc agt gag gac ctc cca ggg cct ggt ggc agt gtg gac ata gtg gcc      1324
Pro Ser Glu Asp Leu Pro Gly Pro Gly Gly Ser Val Asp Ile Val Ala
                385                 390                 395

atg gat gaa ggc tca gaa gca tcc tcc tgc tca tct gct ttg gcc tcg      1372
Met Asp Glu Gly Ser Glu Ala Ser Ser Cys Ser Ser Ala Leu Ala Ser
            400                 405                 410

aag ccc agc cca gag gga gcc tct gct gcc agc ttt gag tac act atc      1420
Lys Pro Ser Pro Glu Gly Ala Ser Ala Ala Ser Phe Glu Tyr Thr Ile
        415                 420                 425

ctg gac ccc agc tcc cag ctc ttg cgt cca tgg aca ctg tgc cct gag      1468
Leu Asp Pro Ser Ser Gln Leu Leu Arg Pro Trp Thr Leu Cys Pro Glu
    430                 435                 440

ctg ccc cct acc cca ccc cac cta aag tac ctg tac ctt gtg gta tct      1516
Leu Pro Pro Thr Pro Pro His Leu Lys Tyr Leu Tyr Leu Val Val Ser
445                 450                 455                 460

gac tct ggc atc tca act gac tac agc tca ggg gac tcc cag gga gcc      1564
Asp Ser Gly Ile Ser Thr Asp Tyr Ser Ser Gly Asp Ser Gln Gly Ala
                465                 470                 475

caa ggg ggc tta tcc gat ggc ccc tac tcc aac cct tat gag aac agc      1612
Gln Gly Gly Leu Ser Asp Gly Pro Tyr Ser Asn Pro Tyr Glu Asn Ser
            480                 485                 490

ctt atc cca gcc gct gag cct ctg ccc ccc agc tat gtg gct tgc tct      1660
Leu Ile Pro Ala Ala Glu Pro Leu Pro Pro Ser Tyr Val Ala Cys Ser
        495                 500                 505

tag gacaccaggc tgcagatgat cagggatcca atatgactca gagaaccagt           1713

gcagactcaa gacttatgga acagggatgg cgaggcctct ctcaggagca ggggcattgc    1773

tgattttgtc tgcccaatcc atcctgctca ggaaaccaca accttgcagt atttttaaat    1833

atgtatagtt tttttg                                                    1849


<210>  5
<211>  508
<212>  PRT
<213>  Homo sapiens

<400>  5

Met Asp His Leu Gly Ala Ser Leu Trp Pro Gln Val Gly Ser Leu Cys
1               5                   10                  15


Leu Leu Leu Ala Gly Ala Ala Trp Ala Pro Pro Pro Asn Leu Pro Asp
            20                  25                  30


Pro Lys Phe Glu Ser Lys Ala Ala Leu Leu Ala Ala Arg Gly Pro Glu
        35                  40                  45

Glu Leu Leu Cys Phe Thr Glu Arg Leu Glu Asp Leu Val Cys Phe Trp
    50                  55                  60

Glu Glu Ala Ala Ser Ala Gly Val Gly Pro Gly Asn Tyr Ser Phe Ser
65                  70                  75                  80

Tyr Gln Leu Glu Asp Glu Pro Trp Lys Leu Cys Arg Leu His Gln Ala
                85                  90                  95

Pro Thr Ala Arg Gly Ala Val Arg Phe Trp Cys Ser Leu Pro Thr Ala
            100                 105                 110

Asp Thr Ser Ser Phe Val Pro Leu Glu Leu Arg Val Thr Ala Ala Ser
        115                 120                 125

Gly Ala Pro Arg Tyr His Arg Val Ile His Ile Asn Glu Val Val Leu
    130                 135                 140

Leu Asp Ala Pro Val Gly Leu Val Ala Arg Leu Ala Asp Glu Ser Gly
145                 150                 155                 160

His Val Val Leu Arg Trp Leu Pro Pro Pro Glu Thr Pro Met Thr Ser
                165                 170                 175

His Ile Arg Tyr Glu Val Asp Val Ser Ala Gly Asn Gly Ala Gly Ser
            180                 185                 190

Val Gln Arg Val Glu Ile Leu Glu Gly Arg Thr Glu Cys Val Leu Ser
        195                 200                 205

Asn Leu Arg Gly Arg Thr Arg Tyr Thr Phe Ala Val Arg Ala Arg Met
    210                 215                 220

Ala Glu Pro Ser Phe Gly Gly Phe Trp Ser Ala Trp Ser Glu Pro Val
225                 230                 235                 240

Ser Leu Leu Thr Pro Ser Asp Leu Asp Pro Leu Ile Leu Thr Leu Ser
                245                 250                 255

Leu Ile Leu Val Val Ile Leu Val Leu Leu Thr Val Leu Ala Leu Leu
            260                 265                 270

Ser His Arg Arg Ala Leu Lys Gln Lys Ile Trp Pro Gly Ile Pro Ser
        275                 280                 285

Pro Glu Ser Glu Phe Glu Gly Leu Phe Thr Thr His Lys Gly Asn Phe
    290                 295                 300

Gln Leu Trp Leu Tyr Gln Asn Asp Gly Cys Leu Trp Trp Ser Pro Cys
305                 310                 315                 320

Thr Pro Phe Thr Glu Asp Pro Pro Ala Ser Leu Glu Val Leu Ser Glu
                325                 330                 335

Arg Cys Trp Gly Thr Met Gln Ala Val Glu Pro Gly Thr Asp Asp Glu
            340                 345                 350

Gly Pro Leu Leu Glu Pro Val Gly Ser Glu His Ala Gln Asp Thr Tyr
        355                 360                 365

Leu Val Leu Asp Lys Trp Leu Leu Pro Arg Asn Pro Pro Ser Glu Asp
    370                 375                 380

Leu Pro Gly Pro Gly Gly Ser Val Asp Ile Val Ala Met Asp Glu Gly
385                 390                 395                 400

Ser Glu Ala Ser Ser Cys Ser Ser Ala Leu Ala Ser Lys Pro Ser Pro
                405                 410                 415

Glu Gly Ala Ser Ala Ala Ser Phe Glu Tyr Thr Ile Leu Asp Pro Ser
            420                 425                 430

Ser Gln Leu Leu Arg Pro Trp Thr Leu Cys Pro Glu Leu Pro Pro Thr
        435                 440                 445

Pro Pro His Leu Lys Tyr Leu Tyr Leu Val Val Ser Asp Ser Gly Ile
    450                 455                 460

Ser Thr Asp Tyr Ser Ser Gly Asp Ser Gln Gly Ala Gln Gly Gly Leu
465                 470                 475                 480

Ser Asp Gly Pro Tyr Ser Asn Pro Tyr Glu Asn Ser Leu Ile Pro Ala
                485                 490                 495

Ala Glu Pro Leu Pro Pro Ser Tyr Val Ala Cys Ser
            500                 505

<210>  6
<211>  1527
<212>  DNA
<213>  Homo sapiens

<400>  6
atggaccacc tcggggcgtc cctctggccc caggtcggct ccctttgtct cctgctcgct      60

ggggccgcct gggcgccccc gcctaacctc ccggacccca agttcgagag caaagcggcc     120

ttgctggcgg cccgggggcc cgaagagctt ctgtgcttca ccgagcggtt ggaggacttg     180

gtgtgtttct gggaggaagc ggcgagcgct ggggtgggcc cgggcaacta cagcttctcc     240

taccagctcg aggatgagcc atggaagctg tgtcgcctgc accaggctcc cacggctcgt     300

ggtgcggtgc gcttctggtg ttcgctgcct acagccgaca cgtcgagctt cgtgccccta     360

gagttgcgcg tcacagcagc ctccggcgct ccgcgatatc accgtgtcat ccacatcaat     420

gaagtagtgc tcctagacgc ccccgtgggg ctggtggcgc ggttggctga cgagagcggc     480

cacgtagtgt tgcgctggct cccgccgcct gagacaccca tgacgtctca catccgctac     540

gaggtggacg tctcggccgg caacggcgca gggagcgtac agagggtgga gatcctggag     600

ggccgcaccg agtgtgtgct gagcaacctg cggggccgga cgcgctacac cttcgccgtc     660

cgcgcgcgta tggctgagcc gagcttcggc ggcttctgga gcgcctggtc ggagcctgtg     720

tcgctgctga cgcctagcga cctggacccc ctcatcctga cgctctccct catcctcgtg     780

gtcatcctgg tgctgctgac cgtgctcgcg ctgctctccc accgccgggc tctgaagcag     840

aagatctggc ctggcatccc gagcccagag agcgagtttg aaggcctctt caccacccac     900

aagggtaact tccagctgtg gctgtaccag aatgatggct gcctgtggtg gagcccctgc     960

acccccttca cggaggaccc acctgcttcc ctggaagtcc tctcagagcg ctgctggggg    1020

acgatgcagg cagtggagcc ggggacagat gatgagggcc ccctgctgga gccagtgggc    1080

agtgagcatg cccaggatac ctatctggtg ctggacaaat ggttgctgcc ccggaacccg    1140

cccagtgagg acctcccagg gcctggtggc agtgtggaca tagtggccat ggatgaaggc    1200

tcagaagcat cctcctgctc atctgctttg gcctcgaagc ccagcccaga gggagcctct    1260

gctgccagct ttgagtacac tatcctggac cccagctccc agctcttgcg tccatggaca    1320

ctgtgccctg agctgccccc taccccaccc cacctaaagt acctgtacct tgtggtatct    1380

gactctggca tctcaactga ctacagctca ggggactccc agggagccca agggggctta    1440

tccgatggcc cctactccaa cccttatgag aacagcctta tcccagccgc tgagcctctg    1500

ccccccagct atgtggcttg ctcttag                                        1527